Europäisches Patentamt

European Patent Office

Office européen des brevets

(19)

(11) Publication number: **0 504 977 A1**

# EUROPEAN PATENT APPLICATION

(21) Application number: 92200686.1

(22) Date of filing: 11.03.92

(51) Int. Cl.5: **A61M 16/00**

Priority: 220391 IT MI9100073.

(30) Priority: **22.03.91**

(43) Date of publication of application:
**23.09.92 Bulletin  92/39**

(84) Designated Contracting States:
**CH DE ES FR GB IT LI**

(71) Applicant: **KONTRON INSTRUMENTS HOLDING N.V.**

Julianaplein 22
**Willemstad, Curacao(AN)**

(72) Inventor: **Damia, Giorgio**
**Via Melzi d'Eril, 18**
**I-20154 Milano(IT)**

(74) Representative: **Riccardi, Sergio**
**Riccardi & Co. Via Macedonio Melloni, 32**
**I-20129 Milano(IT)**

(54) Method and device for reduction of pulmonary tidal volume for mechanical pulmonary ventilators.

(57) A method and device is described which allow normal pulmonary ventilators for adults to be used also for children and newborn babies, since it allows the pulmonary tidal volume supplied by normal ventilators to be reduced by means of an accessory pressure chamber (C) fed by gaseous fluid supplied by the ventilator, on the inside of which there is a squeezable reservoir balloon (P), which receives and supplies the patient with anaesthetic gases at a pressure and in a volumetric quantity per unit of time lower than that supplied by said ventilator.

This invention refers to a method and device for the reduction of the pulmonary tidal volume, to be applied to any mechanical pulmonary ventilator for adults, in order to use it on children and newborn babies.

The device according to this invention can be considered an accessory of modern anaesthetic equipment for the administration of a general anaesthetic.

Modern anaesthetic equipment classically is made up of three parts:

(1) Group for the preparation of the gas mixtures and volatile narcotics to make the patient respire and for their respective doses;

(2) Respiratory circuits composed of tubes, reservoir balloons, valves of various types through which the gaseous anaesthetic mixture for the patients to respire is conducted. The circuit is applied to the air passages by means of a airtight mask or a tube with pneumatic sleeve introduced directly in the trachea. If the circuit has a reservoir balloon, as is almost always the case, the anaesthetist can rhythmically compress the balloon by hand, carrying out an efficient artificial respiration;

(3) mechanical pulmonary ventilator. Today it is an essential part of the anaesthetic equipment, in as much as both for the depressive effect of narcotics and the use of curares during anaesthesia, the respiration of the patient is always insufficient. For these reasons general anaesthesia today is partially or totally carried out by artificial respiration.

The basic pulmonary ventilating performance of a conventional mechanical pulmonary ventilator, is identified as insufflation in the lungs of a gaseous volume known as the tidal volume for a certain number of times per minute and overcoming resistance of various types. In a normal adult man the tidal volume varies from 400 to 600 millilitres (ml) and the frequency per minute from 12 to 16.

It is however evident that in the course of a lifetime there are considerable variations in these values, in particular as a function of age. A newborn baby has a tidal volume of 30-50 ml and a frequency of 30-40. A severely premature baby may even have a tidal volume of 10-15 ml and a frequency of 50-60.

Intermediate values are observed during growth. It is therefore necessary to provide apparatus which can be adapted to these needs, but it is clearly difficult for a single machine to provide for the whole range of these performances. However already for some time respiratory circuits have undergone adaptations and the industry has miniaturised circuits and accessories to adapt them for different ages, but it concerned mostly tubing, connections, balloons, masks and tracheal tubes.

When one wanted to pass to artificial mechanical ventilation usually this was not possible with a normal ventilator, given the oversizing of the spaces of the normal ventilator for adults.

For these reasons international regulations require that three types of ventilator are available: adult, paediatric, and for newborn babies, but it is evident that for hospitals which only occasionally operate on children and newborn babies, the availability of a ventilator for children and newborn babies certainly represents a practical complication as well as a not indifferent expense.

The apparatus according to the present invention, of relatively low cost and volume and simple to use, fills this large gap and may in practice be utilised with all ventilators set up on anaesthetic apparatus since it has a broad frequency range. It may in fact provide tidal volumes with a good precision varying between 15 and 250 ml.

The method according to the present invention substantially consists of using the gaseous fluid provided by conventional pulmonary ventilators to ventilate an accessory pressure chamber with a reservoir balloon inside which receives and supplies the patient with anaesthetic gases at a pressure and in a volumetric quantity per unit of time lower than that supplied by the ventilator, in order that the reservoir balloon will feed the anaesthetic gases at the desired tidal volume and frequency according to the patient to be treated.

The apparatus suitable for carrying out said method is characterised by the features indicated in the claims and described in detail in the following description of its preferred form of embodiment, shown in the appended sheet of drawings, which is an illustrative scheme of the device, its individual component elements may obviously have forms more suitable for producing them in any material fit for the purpose.

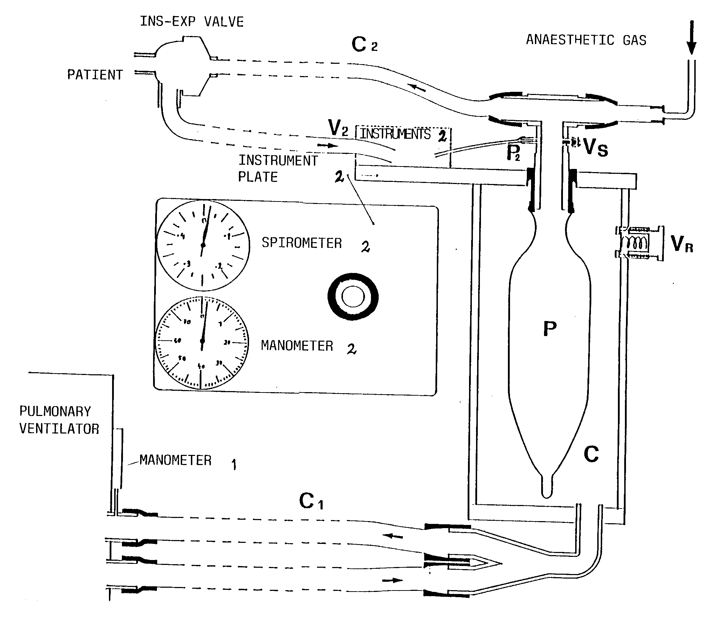

The device according to the present invention is schematically represented in the figure of the attached sheet of drawings and comprises a transparent airtight chamber (C) with rigid walls, in which there is a balloon of rubbery material (P) with thin and flaccid walls. The volume of the chamber is about 1000 ml. The balloon when full, but not overstretched, has a capacity of 500 ml, but for functional reasons must not supply tidal volumes greater than 250 ml. The chamber operates in a vertical position. The neck of the balloon protrudes from the top of the chamber by means of an airtight hole. The balloon is connected to a respiratory circuit of a paediatric-newborn type (C2); the whole paediatric circuit may be detached from the chamber by simply extracting the balloon which seals the exit hole by means of a rubber connection incorporated in the neck of the balloon. This simplifies the changing of the circuit which in our case may also be made in a disposable single use mode.

Through an entrance of internal diameter 12 mm, the chamber is connected to the patient connecting means of a circuit (C1) of a normal ventilator for anaesthetising adults. In other words instead of ventilating the patient's lungs it ventilates the chamber of the reducing device. The pressure in said chamber is thus intermittent and presses against the walls of the balloon sending the gas contained in the balloon towards the lungs of the little patient. The balloon is re-supplied with the narcotic gases prepared by the anaesthetic apparatus while the ventilator according to its configuration will use either environmental air if it is designed for said use, or, if not, oxygen or compressed air coming from a simple supply. Good precision in the supply of the volume which ventilates the chamber of the device is not indispensable. In fact it is sufficient to supply a volume of 3000 - 4000 ml per minute to satisfy the requirements. This volume must be greater than the volumes which are needed to ventilate the patient. To achieve this purpose the balloon must be well squeezed and emptied for each action, thus the necessity for a chamber with good transparency to observe the phenomenon. The pressure in the chamber depends on a regulation valve with external output (VR) that the anaesthetist adjusts each time by reading the pressure on the manometer 1 of the ventilator equipment. There is a another safety valve (Vs) on patient circuit (C2) to prevent the pressure exceeding 35 centimetres of water in the patient circuit.

The circuit which goes to the patient is a linear circuit in which the anaesthetic gases enter in a continuous flow. Near to the air passages of the patient there is a classic inspiratory-expiratory valve which divides the inhaled gases from the exhaled gases. It is indispensable that the distributers which measure the anaesthetic gases supplied are precise and thus also the frequency established on the ventilator is precise. In fact the tidal volume which will ventilate the patient will depend on this precision. For safety the device comprises a manometer (2) and a spirometer (2) the former measuring the pressure (P2) in the circuit (C2), the second measuring the tidal volume returning from the air passages of the patient (V2) to check for any losses in the patient circuit or discrepancies between the volume which has ventilated the lungs and that supplied by the anaesthetic apparatus and entering the balloon.

The device according to this invention including the particular paediatric-newborn circuit may be produced with a simple accessory group, suitable for installing with the maximum ease to any mechanical pulmonary ventilator for adults, and also connected easily to its instrumentation and to the respiratory circuit of normal ventilatory equipment.

For a more complete understanding and illustration of the method of the apparatus according to this invention, the method of operation to be followed in the use of the said apparatus is now described in detail.

(1) The tidal volume and frequency per minute, is measured for a given child, for example 50 ml and 30 per minute. The volume per minute is calculated to be 1500 ml.

(2) The pulmonary ventilator of the anaesthetic apparatus is set to the choosen frequency.

(3) The volume per minute which will ventilate the patient in general made up of a gaseous phase of a mixture of nitrogen protoxide and oxygen, is obtained by regulating the gases entering the patient circuit (C2) precisely on the flowmeter of the anaesthetic apparatus.

(4) The ventilation of the pulmonary ventilator is regulated to a volume per minute which is a little higher than the 1500 ml predetermined for the patient circuit. One must note that in the pulmonary ventilators in adult anaesthetic apparatus, the minimum volume per minute which can be supplied is about 2500 ml. The circuit of the ventilator (C1) is connected to the chamber of the reducing device and the output valve (VR) is opened. The ventilator is switched on. By progressively closing the valve VR, the peak pressure of the ventilator is obtained, which can be read on manometer (1), present on all mechanical ventilators, at about 20 cm of water.

(5) The circuit (C2) which already receives the anaesthetic gases is connected to the intubated patient, inserting a heat and humidity exchange between the inspiratory-expiratory valve and the tracheal tube for children whose internal volume must be taken into account in calculating the pre-set tidal volume.

(6) Having started the pulmonary ventilation it is checked that the balloon is well squeezed at each action. The pressure on the manometer 1 will always be greater than that shown by the manometer 2; if the difference is greater than 3-5 cm of water, it should be lowered by means of valve VR, however always taking care that the balloon P is completely emptied. In the event that the balloon does not empty well, one must close the valve VR slowly until emptying is achieved. The volume ventilated is indicated by the spirometer 2; both the ventilated volume and the volume per minute supplied will be checked. An obvious difference indicates a loss in circuit 2 to be found and eliminated; a small difference may depend on an imprecision of the flowmeter, in which case, for safety it will be better to increase the supply to bring the volume indicated on the spirometer 2 to the estimated value.

From the previous detailed description one can thus see that the above described and illustrated method and device fully realise the predetermined objects and constitute very important progress solving a technical problem much felt in the field of apparatus for anaesthesia, but one must also understand that considerable modifications, variations, additions and/or substitutions of elements which carry out the same operative functions may be made, without thus leaving either the spirit nor the object of the invention and without leaving its scope of protection as results from the appended claims.

## Claims

1. Method for the reduction of the pulmonary tidal volume for application to mechanical pulmonary ventilators, characterized in that they consist essentially of utilising gaseous fluid supplied by conventional pulmonary ventilators, to ventilate an accessory pressure chamber at the inside of which a reservoir balloon is provided which receives and supplies anaesthetic gases to the patient at a pressure and in a volumetric quantity per unit of time lower than that supplied by the ventilator, in order that the reservoir balloon will feed the anaesthetic gases at the desired tidal volume and frequency according to the patient to be treated.

2. Method according to claim 1, characterized in that the pressure and volume ventilated to the patient are controlled by means of suitable instruments applied to the circuit supplying anaesthetic gases to the patient.

3. Method according to claim 1, characterized in that the pressure in the accessory chamber is regulated by means of an external output adjusting valve, according to the pressure supplied by the ventilator and controlled on the manometer of this apparatus.

4. Device for the reduction of pulmonary tidal volume, which can be applied to mechanical pulmonary ventilators for adults in order to use them on children or newborn babies, characterized in that it consists essentially of an accessory pressure chamber, fed by a gaseous fluid supplied by a conventional pulmonary ventilator, at the inside of which a crushable reservoir balloon is provided, which receives and supplies anaesthetic gases to the patient at a pressure and in a volumetric quantity per unit of time lower than that supplied by the ventilator, in order that the reservoir balloon will feed the anaesthetic gas at the desired tidal volume and frequency according to the patient to be treated.

5. Device according to claim 4, characterized in that the accessory pressure chamber is made of transparent material to check that the reservoir balloon is well squeezed.

6. Device according to claim 4, characterized in that the accessory chamber has a valve for regulating the pressure by external output.

7. Device according to claim 4, characterized in that it also comprises a spirometer and a manometer for controlling the ventilated volume and the pressure in the circuit supplying anaesthetic gases to the patient.

8. Device according to claim 4, characterized in that it comprises a safety valve on the patient circuit to prevent the pressure exceeding the pre-set value.

9. Device according to claim 4, characterized in that the patient circuit is of a paediatric linear type and can be detached from the accessory chamber and is adapted for a disposable single use mode.

10. Device for the reduction of the pulmonary tidal volume, which can be applied to pulmonary mechanical ventilators, substantially as described hereinbefore and as shown in the appended illustrative drawing, for the above specified objects.

INS-EXP VALVE

ANAESTHETIC GAS

$C_2$

PATIENT

$V_2$ INSTRUMENTS 2

INSTRUMENT
PLATE

$P_2$

$V_S$

2

$V_R$

SPIROMETER 2

MANOMETER 2

P

PULMONARY
VENTILATOR

MANOMETER 1

C

$C_1$

EP 0 504 977 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | EP-A-0 166 305 (GAMBRO AB) <br> * page 4, line 11 - line 20 * <br> * page 6, line 12 - line 26 * <br> * page 7, line 11 - line 18 * <br> * figures * <br> --- | 1-10 | A61M16/00 |
| Y | FR-A-1 479 738 (BLEASE LTD) <br> * the whole document * <br> --- | 1-10 | |
| A | US-A-4 883 051 (WESTENSKOW ET AL.) <br> * claim 1; figures 1,2,6 * <br> --- | 1 | |
| A | US-A-3 918 447 (INKSTER ET AL.) <br><br> ----- | | |
| | | | TECHNICAL FIELDS SEARCHED (Int. Cl.5 ) |
| | | | A61M |

The present search report has been drawn up for all claims

| Place of search | Date of completion of the search | Examiner |
|---|---|---|
| THE HAGUE | 15 JUNE 1992 | VILLENEUVE J.M. |

EPO FORM 1503 03.82 (P0401)